# EUROPEAN PATENT SPECIFICATION

(11) **EP 1 709 183 B1**
(45) Date of publication and mention of the grant of the patent: **12.05.2010**
(21) Application number: 04816631.8
(22) Date of filing: 10.12.2004
(51) Int. Cl.: C12N 15/82, A01H 5/00, A01H 3/00

(54) **METHOD OF MODIFYING PLANT PHENOTYPES WITH NONSYMBIOTIC HEMOGLOBIN**
VERFAHREN ZUM MODIFIZIEREN PFLANZLICHER PHÄNOTYPEN MIT NICHTSYMBIOTISCHEM HÄMOGLOBIN
METHODE POUR MODIFIER DES PHENOTYPES VEGETAUX AVEC DE L'HEMOGLOBINE NON SYMBIOTIQUE

(30) Priority: 12.12.2003 US 528777 P
(43) Date of publication of application: 11.10.2006
(73) Proprietor: University of Manitoba, Manitoba R3T 5V4 (CA)
(72) Inventor: HILL, Robert, D., Winnipeg, Manitoba R2M 5H4 (CA); BARON, Kevin, Carberry, Manitoba R0K 0H0 (CA)
(74) Representative: Plougmann & Vingtoft A/S
(86) International application number: PCT/IB2004/004419
(87) International publication number: WO 2005/055703

(56) References cited:
- WO-A-99/02687
- WO-A-2004/057946
- WO-A1-98/12913
- WO-A2-00/00597
- WO-A2-2004/087755
- US-B1- 6 372 961
- SEREGELYES CSABA ET AL: "Phytoglobins can interfere with nitric oxide functions during plant growth and pathogenic responses: A transgenic approach." PLANT SCIENCE (OXFORD), vol. 165, no. 3, September 2003 (2003-09), pages 541-550, XP003001105 ISSN: 0168-9452
- DORDAS C ET AL: "Expression of a stress-induced hemoglobin affects NO levels produced by alfalfa root cultures under hypoxic stress" PLANT JOURNAL, BLACKWELL SCIENTIFIC PUBLICATIONS, OXFORD, GB, vol. 35, September 2003 (2003-09), pages 763-770, XP003001104 ISSN: 0960-7412
- HÄGGMAN, H., ET AL.: "expression of Vitreoscilla haemoglobin in hybrid aspen (Populus tremula x tremuloides)" PLANT BIOTECHNOLOGY JOURNAL, vol. 1, July 2003 (2003-07), pages 287-300, XP002470469 blackwell publishing Ltd
- HEBELSTRUP KIM H ET AL: "Metabolic effects of hemoglobin gene expression in plants" GENE (AMSTERDAM), vol. 398, no. 1-2, Sp. Iss. SI, August 2007 (2007-08), pages 86-93, XP002470470 ISSN: 0378-1119
- HUNT P.W. ET AL: 'Increased levelof hemoglobin 1 enhances survival of hypoxic stress and promotes early growth in Arabidopsis thaliana' PROC. NATL. ACAD SCI. USA vol. 99, no. 26, 24 December 2002, pages 17197 - 17202, XP008071042
- IGAMBERDIEV A.U. ET AL: 'NADH-dependent metabolism of nitric oxide in alfalfa root cultures expressing barley hemoglobin' PLANTA vol. 219, May 2004, pages 95 - 102, XP008071016
- HOLMBERG N. ET AL: 'Transgenic tobacco expressing Vitreoscilla hemoglobin exhibits enhanced growth and altered metabolite production' NATURE BIOTECHNOLOGY vol. 15, no. 3, 1997, pages 244 - 247, XP002075761

## Description

### BACKGROUND OF THE INVENTION

The present invention relates generally to the field of agriculture.

Hemoglobins are widespread throughout the biosphere. *(See* Wittenberg and Wittenberg,1990, Ann. Rev Biophys Chem. 19:217-241). They are found in a broad range of organisms from bacteria, through unicellular eukaryotes, to plants and animals, suggesting that they predate divergence of life into plant and animal forms.

Plant hemoglobins have been classified into symbiotic and nonsymbiotic types (Appleby, 1992, Sci Progress 76:65-398). Symbiotic hemoglobins are found in plants that are capable of participating in microbial symbioses, where they function in regulating oxygen supply to nitrogen fixing bacteria. Nonsymbiotic hemoglobins were discovered recently discovered and are thought to be the evolutionary predecessors of the more specialized symbiotic leghemoglobins. The ubiquitous nature of nonsymbiotic hemoglobins is evidenced by their broad presence across the plant kingdom. (*See* Appleby, 1985, Nitrogen Fixation and CO2 Metabolism, eds. Ludden and Burris, pp. 41-51).

The widespread presence and long evolutionary history of plant hemoglobins suggest a major role for them in the life of plants. Nonsymbiotic plant hemoglobins (nsHb), consisting of class 1, class 2, and truncated Hbs (class 3) are believed to be expressed universally in members of the plant kingdom. (Andersson et al, 1996, Proc Natl Acad Sci 93: 5682 -5687; Watts et al, 2001, PNAS 98: 10119 -10124).

The existence of plant hemoglobins in the root nodules of legumes for almost has been known for almost 60 years. (*See, e.g.,* Kubo, 1939, Acta Phitochem 11:195-200; Keilen and Wang, 1945, Nature 155:227-229). Over the years, hemoglobins have been positively identified in three non-leguminous dicotyledonous plants: *Parasponia andersonii, Tream tomentosa,* and *Casuarina glauce*. (*See, e.g.,* Appleby et al., 1983, Science 220:951-954; Bogusz et al., 1988, Nature 331:178-180; Kortt et al., 1988, FEBS Lett 180:55-60). Recently, an Hb cDNA from barley was isolated and the gene was demonstrated to be expressed in seed and root tissues under anaerobic conditions. (*See* Taylor et al., 1984, Plant Mol Biol 24:853-882). These observations support the viewpoint that plant hemoglobins have a common origin. *(See* Landsmann et al., 1986, Nature 324:166-168). Since Hb has been demonstrated to occur in two of the major divisions of the plant kingdom, it is likely that an Hb gene is present in the genome of all higher plants. *(See* Brown et al., 1984, J Mol Evol 21:19-32; Bogusz et al., 1988; Appleby, 1992, Sci Progress 76:365-398; Taylor et al., 1994, Plant Mol Biol 24: 853-862; Andersson et al., 1996, Proc Natl Acad Sci USA 93:427431; Hardison, 1996, Proc Natl Acad Sci USA 93:5675-5682).

The reported lack of effect of hemoglobin on cell growth and oxygen uptake under normal air conditions likely reflects the fact that barley *(See* Taylor et al., 1994, Plant Mol Biol 24: 853-862) and maize hemoglobin genes are induced under conditions of limited oxygen availability, resulting in the protein having little effect when oxygen supplies are not impaired. It has been shown clearly that the energy status of maize cells when oxygen is limiting is affected by the ability of the cells to produce hemoglobin. Total adenylates and ATP levels are maintained during the period of exposure to limiting oxygen when hemoglobin is constitutively expressed in the cells. *(See* WO 00/00597). Alternatively, when hemoglobin expression was suppressed by constitutive expression of antisense barley hemoglobin message, the cells were unable to maintain their energy status during oxygen limitation.

Class 1 nonsymbiotic hemoglobins are present in seed, root and stem tissue of monocots and dicots where they are expressed in response to hypoxia, etiolation, sucrose/mannitol addition, cytokinin, ARR1 or auxin (IAA) treatments in addition to nutrient oversupply (NO₃⁻, NO₂⁻ and NO) and deprivation (P, K, and Fe). (*See* Taylor et al., 1994, Plant Mol Biol 24: 853-862; Hunt et al, 2001, Plant Mol Biol 47: 677 - 692; Lira-Ruan et al, 2001, Plant Sci 161: 279 -287; Kim et al., 2003, Journal of Plant Biology 46: 161-166; Ohwaki et al., 2003, Plant and Cell Physiology 44: S78; Ross et al, 2004, J Exp Bot 55: 1721 -1731; Wang et al, 2003, Plant Cell Environ 26: 673 -680; Dordas et al, 2003, Plant Journal 35: 763 -770). Class 1 nsHbs are also known to be repressed in roots following infection by mycorrhizal fungi. *(See* Uchiumi et al, 2002, Plant Cell Physiol 43: 1351 -1358).

Hunt et al., 2002, PNAS 99: 17197-202, reported that *A. thaliana* over-expressing a class 1 *A. thaliana* nsHb (GLB1-high affinity) showed improved survival following severe hypoxic stress, and that similar *A. thaliana* plants transformed to over-express *Parasponia* class 1 Hb (GLB1 S-medium affinity) demonstrated an intermediate level of hypoxic protection relative to controls and to plants transformed with GLB1 mutated to have a low affinity for gaseous ligands (GLB1(HE7L)-low affinity).

More recent work with transgenic maize cell suspensions (Dordas et al, 2004, Planta 219: 66 -72), alfalfa root cultures (Dordas et al, 2003, Plant Journal 35: 763-770; Igamberdiev et al, 2004, Planta 219: 95 -102) and *A. thaliana* plants (Perazzolli et al, 2004, Plant Cell 16: 2785 -2794) has demonstrated that class 1 nsHbs modulate plant NO levels, both *in vitro* and *in vivo,* with NO levels being inversely related to class 1 nsHb expression. Both barley and alfalfa class 1 nsHbs, together with a corresponding reductase, have been shown to metabolize NO to NO₃⁻, with such activity being NAD(P)H-dependent and displaying characteristics of a NO-dioxygenase. *(See* Igamberdiev et al., *supra;* Seregelyes et al, 2004, FEBS Lett 571: 61 -66).

Transgenic tobacco (*Nicotiana tabacum*) plants expressing a hypoxia-inducible bacterial hemoglobin (VHb) from the obligate aerobic, gram-negative bacteria *Vitreoscilla* have been shown to exhibit reduced emergence time, enhanced growth, accelerated development and increased chlorophyll content relative to control plants (Holmberg et al. 1997, Nature Biotechnol 15: 244-247). Petunias (*Petunia hybrida*) and tobacco plants expressing VHb also have demonstrated improved hydroponic growth and waterlogging tolerance relative to control plants. (*See* Mao et al. 2003, Acta Botanica Sinica 45: 205-210). VHb is a bacterial hemoglobin, and is separate and distinct from the plant nonsymbiotic hemoglobins encompassed by the present invention. For example, VHb has different biochemical properties than nsHb, has different ligand-binding properties, and has a lower oxygen affinity.

Hunt *et al.,* 2002, *supra,* reported that that GLB1-transformed plants exhibited increased early growth (*i.e*., at 14 days), greater root and shoot weight at 14 days, and had longer roots with a lower root hair density and more lateral roots than control plants, when the transformed plants were grown under normal oxygen conditions. However, no altered development rate of leaf production was observed. Additionally, Hunt *et al.* found no differences in morphological development of *A. thaliana* plants expressing either *Arabidopsis* or *Parasponia* class 1 nsHb. The authors hypothesized that, although the plant was grown under normoxic conditions, the plant may have experienced localized, transient hypoxia, noting that a transient hypoxic phase may be experienced during germination. They therefore associated the observed effects on early root growth as being due to the transformed plant's improved ability to withstand that hypoxia.

While the effects of nonsymbiotic hemoglobin on oxygen uptake, NO levels, and survival under hypoxic conditions have been studied, the ability to modify plant phenotypes or mineral nutrition under normal oxygen conditions by controlling levels of nonsymbiotic hemoglobin has not heretofore been determined. Indeed, conflicting reports exist as to the influence of class 1 nsHb and/or VHb expression on plant growth under non-stressed, as compared to stressed, conditions. (*See, e.g.,* Seregelyes et al. 2004, Febs Letters 571: 61-66; Haggman et al. 2003, Plant Biotechnology Journal 1: 287-300; Frey et al. 2004; Perazzolli et al. 2004, Plant Cell 16: 2785-2794).

There are a number of different plant phenotypes that it would be useful to be able to modify. For example, apical dominance in shoots and roots, taproot width, leaf size, leaf length, petiole length, internode length, plant shape, erect versus prostrate growing habit, flower color, early versus late flowering, chlorophyll content, and nutrient uptake, concentration, or metabolism.

### SUMMARY

The invention relates to a method of modifying one or more plant phenotypes by altering the level of non-symbiotic hemoglobin (nsHb) expression in the plant. The method comprises transforming a plant with an expression vector comprising a nucleotide sequence encoding a plant non-symbiotic hemoglobin or an antisense sequence thereto, thereby yielding a transformed plant having an altered level of expression of non-symbiotic plant hemoglobin as compared to a non-transformed control plant that is not transformed to alter the level of expression of non-symbiotic plant hemoglobin,
wherein said transformed plant exhibits, under normal oxygen conditions, a plant phenotype that is modified as compared to said non-transformed control plant,
wherein said phenotype is selected from the group consisting of shoot or root apical dominance; flower color; and chlorophyll content
wherein, when said transformed plane exhibits an increased level of expression of non-symbiotic hemoglobin as compared to said non-transformed control plant, said plant exhibits increased shoot apical dominance or greater root apical dominance under normal oxygen conditions as compared to said non-transformed control plant.

In some embodiments, the transformed plant exhibits an increased level of expression of non-symbiotic hemoglobin as compared to said non-transformed plant. Those embodiments may comprise transforming the plant with an expression system comprising a nucleic acid molecule encoding a plant nonsymbiotic hemoglobin. In other embodiments, the transformed plant exhibits a decreased level of expression of non-symbiotic hemoglobin as compared to said non-transformed plant. Those embodiments may comprise transforming the plant with an expression system comprising an antisense plant nonsymbiotic hemoglobin nucleic acid molecule.

Thus, also disclosed are plants transformed in accordance with these methods, exhibiting a modified phenotype under normal oxygen conditions as compared to a non-transformed plant that is not transformed to alter the level of expression of non-symbiotic plant hemoglobin. The plant exhibits an increased level of expression of non-symbiotic hemoglobin as compared to said non-transformed plant. The plant exhibits a decreased level of expression of non-symbiotic hemoglobin as compared to said non-transformed plant.

These and other aspects of the invention are described in more detail below, and are illustrated in the examples.

### BRIEF DESCRIPTION OF THE DRAWINGS

Figure 1 sets forth data on four transformed alfalfa plant lines at 14, 21, 28, 35 and 63 days after transplantation.
Figure 2 shows the chlorophyll content of four transformed alfalfa lines.
Figure 3 shows the stem weight, leaf weight, reproductive tissue weight and shoot dry weight for four transformed alfalfa plants at 14, 21, 28, 35 and 63 days after transplantation.
Figure 4 is a bar graph of leaf:stem and shoot:root ratios of four transformed alfalfa plants at 28 days after transplantation.

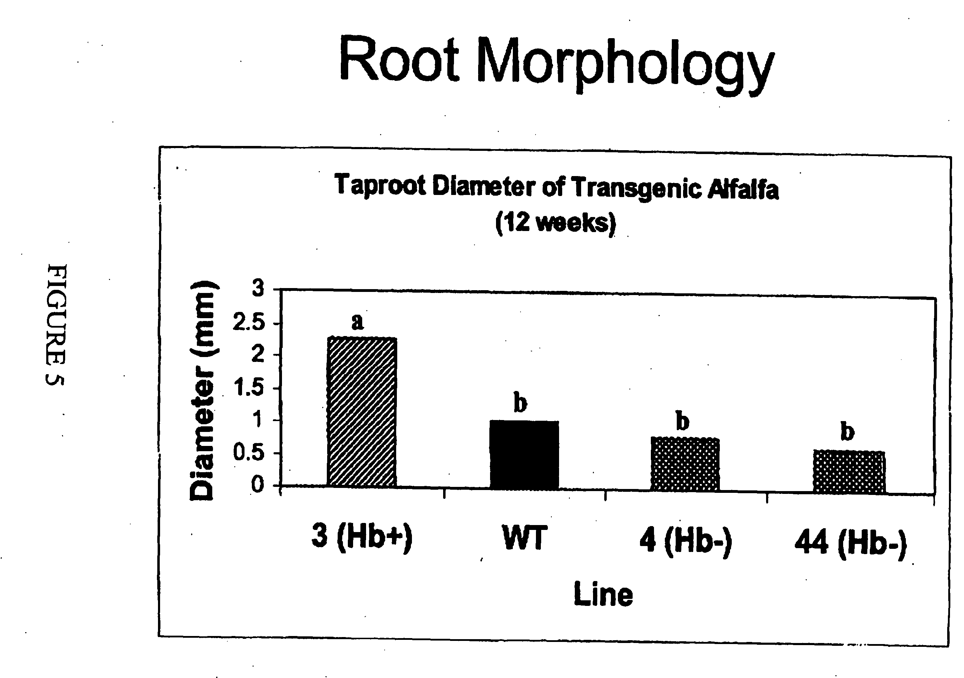

Figure 5 is a bar graph of root morphology of four transformed alfalfa plants at 12 weeks after transplantation.

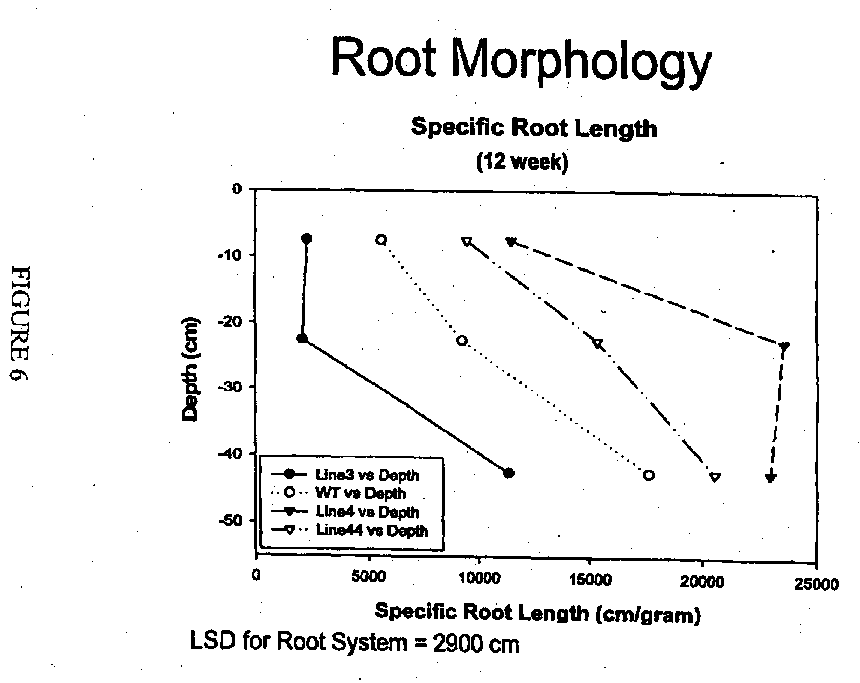

Figure 6 is a comparative graph of depth versus specific root length for four transformed alfalfa plants at 12 weeks after transplantation.

### DESCRIPTION

Described herein are methods of modifying one or more plant phenotypes by altering the level of non-symbiotic hemoglobin (nsHb) expression in the plant. In one aspect, the methods comprise modifying a plant to over-express nsHb, such that the plant exhibits an increased level of nsHb (Hb+) relative to a plant that has not been transformed. In another aspect, the methods comprise modifying a plant to under-express nsHb, such that the plant exhibits a decreased level of nsHb (Hb-) relative to a plant that has not been transformed. In another aspect, only one part of the plant is modified to over-express or under-express nsHb. In another aspect, different parts of the plant (such as the roots or shoots, stems) are independently modified to over-express or under-express nsHb.

The modification can be achieved through standard recombinant technologies. For example, a suitable expression cassette can be integrated into the plant genome.

Alternatively, the plant can be transfected with a suitable expression vector. In one aspect, the expression system (such as the expression cassette or expression vector) comprises a promoter, such as a plant promoter or an inducible or repressible promoter. The use of an inducible or repressible promoter permits selective activation of the nucleotide sequence comprised in the expression vector, so that over-expression or repression of nsHb can be regulated.

In embodiments where over-expression of nsHb is desired, the expression system comprises a nucleotide sequence encoding a plant nsHb. In embodiments where under expression (or repression of nsHb expression) of nsHb is desired, the expression system comprise an antisense nsHb nucleotide sequence, such as an antisense copy of the target plant's nsHb gene.

Also disclosed is the use of any nucleotide sequence encoding a plant nsHb, and the use of antisense sequences thereto. A number of plant nsHb genes have been published. For example, Taylor et al., Plant Mol Biol 24: 853-62 (1994), discloses a barley ns-Hb nucleotide sequence; Arrendondo-Peter, Plant Physiol. 115: 1259-66 (1997) discloses a rice ns-Hb sequence; Trevaskis et al., Proc. Nat'l A cad Sci. USA 94: 12230-34 (1997) describes an *Arabidopsis* ns-Hb sequence; Andersson et al., Proc. Nat'l Acad. Sci. USA 93: 5682-87 (1996)describes a soybean ns-Hb sequence; Jacobsen-Lyon et al., Plant Cell 7: 213-23 (1995), describes an ns-Hb from *Casuarina glauca.* Nonsymbiotic hemoglobin sequences also have been reported for other plants, including *Cichorium* (Hendriks et al., Biochim. Biophys. Acta. 1443: 193-97 (1998), *Lotus japonica* (Uchiumi et al., Plant Cell Physiol. 43: 1351-58 (2002)), wheat and potato (Larsen, Biochim. Biophys. Acta. 1621: 299-305 (2003)), and *Euryale ferox* (Guldner et al., J. Evol. Biol. 17: 48-54 (2004)).

Other methods of achieving over- or under-expression of nsHb also may be used. For example, there are conventional techniques that employ a promoter or repressor to induce or repress expression, respectively, of the plant's nsHb genes.

The expression system may further comprise other components known in the art, such as one or more promoters and one or more selectable markers. In one embodiment, the expression system comprises a strong constitutive promoter. In another embodiment, the expression system comprises a tissue-specific promoter.

In some circumstances, it is advantageous to control expression of the expression cassette or vector at selected points during the plant's life cycle, so as to achieve over-expression or under expression of nsHb at selected time points. This can be achieved by using an expression cassette or vector comprising an inducible or repressible promoter, and by inducing or repressing expression at selected points in the plant's life cycle to achieve the desired effect. Chemical-inducible systems for regulated expression of plant genes are known. *See, e.g.,* Zuo J. & Chua NH, 2000, Curr. Opin. Biotechnol. 11: 146-51. The use of such systems is encompassed by the present invention.

In some circumstances, it is advantageous to effect targeted expression of the expression cassette or vector, so as to achieve over-expression or under expression of nsHb in specific cells, such as root, shoot, stem, leaf or reproductive cells. This can be effected by methods known in the art. For example, the expression cassette or vector may comprise a tissue-specific promoter, such as a root- or shoot-specific promoter. Suitable such promoters are known. *See, e.g.,* Zhang JZ, 2003, Curr Opin Plant Biol 6: 430-440. Alternatively, the expression cassette or vector can be targeted to the target cells, such as, for example, by targeted gene delivery or by direct administration to the target cells.

Any plant can be modified in accordance with the present invention. Exemplary plants include decorative plants such as flowering plants and grasses, forage plants, maize, barley, wheat, wild oat and *Echinochloa crus galli.*

As indicated, plant phenotypes that can be altered in accordance with the invention include apical dominance in shoots and roots, flower color, and chlorophyll content.

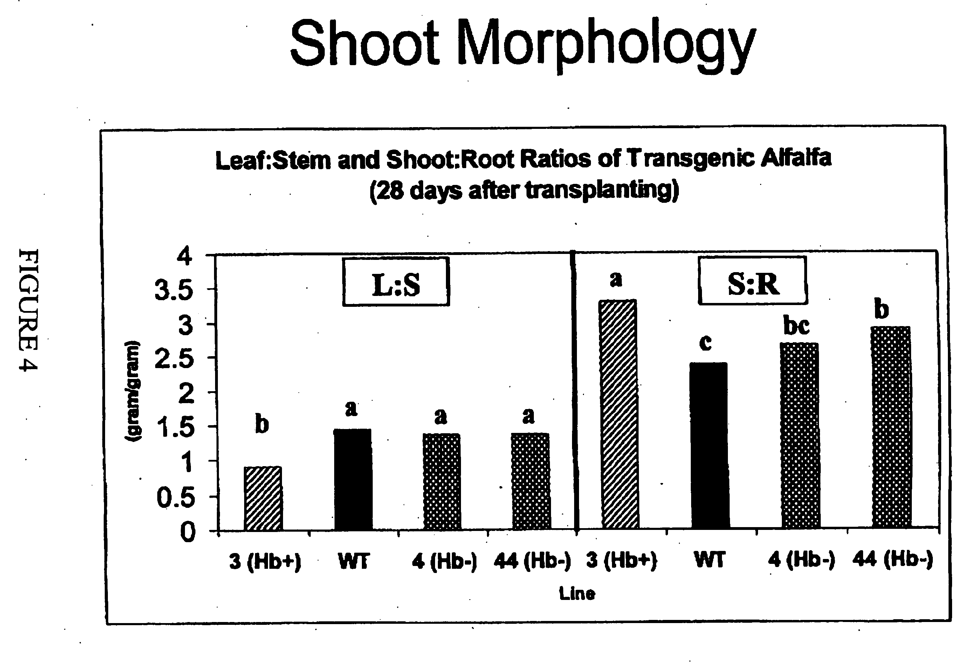

The invention may also make possible ways of modifying the relative proportions of plant components, such as leaf, stem, and reproductive tissue, to enhance seed production or forage quality. Generally, plants modified to over-express nsHb exhibit an increased proportion of reproductive tissue, nsHb+ plants also may exhibit an increased shoot:root ratio and a lower leaf:stem ratio. See Fig. 4. nsHb- plants may exhibit decreased stem yield but increased leaf yield, and thus may exhibit an increased leaf:stem ratio. See Fig. 4.

While not wanting to be bound by any theory, the present inventors believe that the mechanism underlying the above-described changes in phenotype observed when a plant is modified to over-express or under-express nsHb in accordance with the invention, relates to changes in NO levels, which in turn affects hormone expression.

For example, the effects of nsHb over-expression on apical dominance and shoot:root ratios are consistent with an alteration of the auxin:cytokinin ratio; the effects on internode length, petiole length, petiolule length and leaf shapes consistent with an alteration of gibberellin response; the effect on the number of (trifoliate) leaves per node (with nsHb⁺ plants having fewer leaves per node) is consistent with an alteration of a cytokinin response; the effect on branching (with roots and shoots of nsHb- plants showing greater branching) is suggestive of an alteration of auxin:cytokinin ratios, and the effect on flower color also be an alteration of a hormonal response.

In some plants, such as grass, it may be advantageous to modify one phenotype by over-expression of nsHb and one phenotype by under expression of nsHb. For example, grass shoots that under-express nsHb may have a prostrate growing habit that provides good ground coverage. On the other hand, grass roots that over-express nsHb may exhibit apical dominance such that they penetrate the earth more deeply and provide greater drought resistance (separate from the tolerance to hypoxic conditions attributable to nsHb expression itself, as described in WO 00/00597). The present invention provides a method of making a plant modified to express both advantageous phenotypes. Thugs, in accordance with the present invention, the shoots of a plant may be modified to under-express nsHb while the roots of the plant are modified to over-express nsHb.

From the teachings provided herein, those skilled in the art will recognize other combinations of phenotypes for which it may be advantageous to have a plant with one or more phenotypes induced by nsHb- over-expression and one or more phenotypes induced by nsHb under expression.

Other modifications of plant phenotype are demonstrated in the examples set forth below.

### Examples

### Plant Material

Transgenic alfalfa (*Medicago sativa* cv. Regen SY) plants were generated from alfalfa root cultures containing sense and antisense orientation of barley nonsymbiotic hemoglobin as described in Dordas et al., 2003, Plant J 35: 763-70.

### Transformation Vectors

Constructs containing the sense and antisense orientation of barley hemoglobin are obtained from pAS 1 (containing sense) or pAS2 (containing antisense) plasmids. The fragment containing ubiquitin promoter + ubiquitin intron + hemoglobin + nos3' is inserted into the vector pWBVec8. The plasmids are used to transform *A. rhizogenesis* strain A4 using the freeze-thaw method. After transformation, incubation and amplification, plasmids are extracted from the bacteria and restriction enzyme-digested to verify transformation.

### Alfalfa Transformation

Stem segments (2-cm long) are cut from alfalfa plants and placed inverted into Magenta boxes containing MSHF media. A loopful of *A. rhizogenes* A4 containing the appropriate constructs is placed on the exposed end of the explant. The control line (C) is transformed with an empty cassette. After a few weeks, one root from each stem segment is placed on a Petri plate with MSHF media containing 500 mg 1⁼¹ carbenicillin and 20 mg 1⁻¹ hygromycin. Each root is left to grow for few weeks and then screened at the DNA and protein levels for insertion of the hemoglobin gene and for quantification of the levels of expression of hemoglobin.

### Generation of Alfalfa Plants from Alfalfa Root Cultures

Root segments (1 cm) of alfalfa root cultures are cut and placed on Petri Dishes containing SH induction media. After 3 to 4 weeks, root segments develop callus, after which calli are transferred to MSHF medium. Upon formation of somatic embryos, calli are subcultured every two weeks on MSHF medium. In approximately 3-4 months, embryos develop into plantlets. Plantlets are then transferred to magenta boxes containing sterile media and a peat:perlite (1:1) mixture. Following an acclimation period (4 weeks), plantlets are transferred to pots and placed under growth conditions described below.

Four lines were evaluated in these experiments: nsHb⁺ (3), Control (C), nsHb⁻(4) and nsHb⁻ (44). All of the lines evaluated in these studies, including the empty vector control line (C), were generated in a single transformation experiment, as described above. The nsHb content of the four lines studied is shown is Table 1. There is almost ten-fold variation in the nsHb content between the lines having the highest and lowest nsHb content and the relative amounts are consistent with those of the root culture lines (Dordas *et al.,* 2003, *supra*) from which the plants have been regenerated.

**Table 1: nsHb content in the roots of transgenic alfalfa plants (35 DATP)**

| Alfalfa Line | Hemoglobin Content (nmol g⁻¹ Fresh Weight) |
|---|---|
| Hb+ (3) | 12.042a ± 1.384 |
| Control | 4.061b ±.258 |
| Hb- (4) | 1.455c ±.046 |
| Hb- (44) | 1.359c ±.051 |

### Plant Growth Conditions

Maintenance, rooting and growth studies of transgenic alfalfa plants, with the exception of root morphology studies, were conducted in growth chambers (Econaire-GR-36) set at day/night (22/19°C;16h/8h) with relative humidity maintained at 65-85%. Photosynthetic photon flux density within growth chambers ranged from 350 to 500 µmol m⁻² s⁻¹ at pot height. Cuttings of transgenic alfalfa plants were rooted for 20 d in root trainers filled with commercial growth medium (Terra-Lite, 2000; W.R. Grace & Co. Ajax, ON) prior to transplanting into 15-cm diameter pots containing a steam-sterilized sand:soil (2:1; v:v) mixture. Cuttings were initially transplanted at a density of 2 per pot and thinned to 1 per pot 7 days after transplanting ("DATP"). Pots were fertilized once a week with 1g L⁻¹ of a commercial fertilizer (20-20-20, Plant Prod, Brampton, ON, Canada). Within the growth chamber, pots were assigned to quadrats centered about the point of highest light intensity and were rotated weekly to reduce variability in microclimate. Plants in shoot and root morphology experiments were kept well watered for the duration of the experiment(s).

For root morphology studies, stem cuttings (3 per pot) were transplanted into 55 x 20 cm PVC cylinders, placed in the greenhouse, and thinned to 1 per pot 14 DATP. Cylinders were lined with 2.5 cm of gravel and caps drilled to allow free drainage.

Cylinders were then filled with soil mixture as previously described. Temperature was maintained at 25 ± 5 °C for the duration of the experiment. Supplemental lighting was provided by 1000-W high-pressure sodium bulbs, set at 16-h photoperiod and supplying 600-1000 µmol mol m⁻²s⁻¹ of light at pot height. PVC pots were re-randomized within the greenhouse at two-week intervals.

### Harvest Protocols

Leaf, stem, and reproductive characteristics, along with total root and shoot dry weights of transgenic plants were monitored over 63 days of growth with harvests occurring 14, 21, 28, 35, and 63 DATP. For each harvest, shoots were clipped at the junction of the taproot and crown, placed in plastic bags, and held at 4 °C during the 2 days necessary for tissue separation. Shoot samples were separated into leaf, stem, and reproductive fractions. For leaf measurements (14, 21, 28 and 35 DATP) the number of leaves (leaflets plus petiolule) per stem (stem and petiole) was noted prior to leaf area determinations with a leaf area meter (LI-3100, LI-COR Inc., Lincoln, Nebraska, USA). Plants were then separated into individual stems and staged according to the mean stage by count (MSC) method of Kalu and Fick (1981). Stems per plant, nodes per stem, and stem diameter at the centre of the lowermost internode were also recorded. Following shoot harvest, roots were submerged to remove excess rooting medium. Root systems were then placed on a fine screen and a stream of water directed through them. Between the 35 and 63 DATP harvest, time to flowering was noted. Racemes per plant and florets per raceme were recorded on the 72 DATP harvest. All tissues were dried at 70 °C for 48 hours prior to dry weight determinations. At 35 DATP, plants were analyzed for chlorophyll content according to Estill et al. (1991).

### Root Morphology

For harvests occurring 63 and 84 DATP, cylinders were placed horizontally on a fine screen and rooting medium worked away under a stream of water. Following removal of debris, root systems were scored for taproot diameter (TD), lateral root number (LRN), lateral root position (LRP) and determinate taproot position (DTP) according to Johnson et al. (1998). Root systems were then separated into 0-15, 15-30, and 30-55 cm fractions, root lengths recorded using digitizing software (ASSESS software, APS Press), and dry weights determined.

### Nutrient Analysis

For each transgenic line shoot tissue from the 35 DATP harvest and root tissue from 21-35 DATP harvests was ground on a Wiley Mill (1 mm) prior to being analyzed for mineral nutrient composition.

### Experimental Design and Data Analysis

All experiments were analyzed as completely randomized designs. For shoot morphology and root morphology experiments six and three replicates were used respectively. Analysis of variance (SAS Institute, 1985) was used to partition variance into line and replicate effects. Where F-tests were significant, Fisher's protected LSD test (P≤0.05) was used for mean comparisons. Shoot morphology experiments were conducted three times (2x yield and morphology, 1x yield). Seedling root morphology experiments were conducted once while stem cutting root morphology experiments were conducted twice. Data from seedling root morphology experiments has been combined for ease of presentation.

### Example 1: Phenotype. Growth, and Morphology of Transgenic Alfalfa

Transformation of alfalfa plants to over-express (Hb+) or under-express (Hb-) nsHb performed as described above resulted in the following phenotype modifications:

### Flower Color

The modification of nsHb levels resulted in a change in flower color. The intensity of the purple color increased as nsHb expression declined.

### Leaf Greenness & Chlorophyll Content

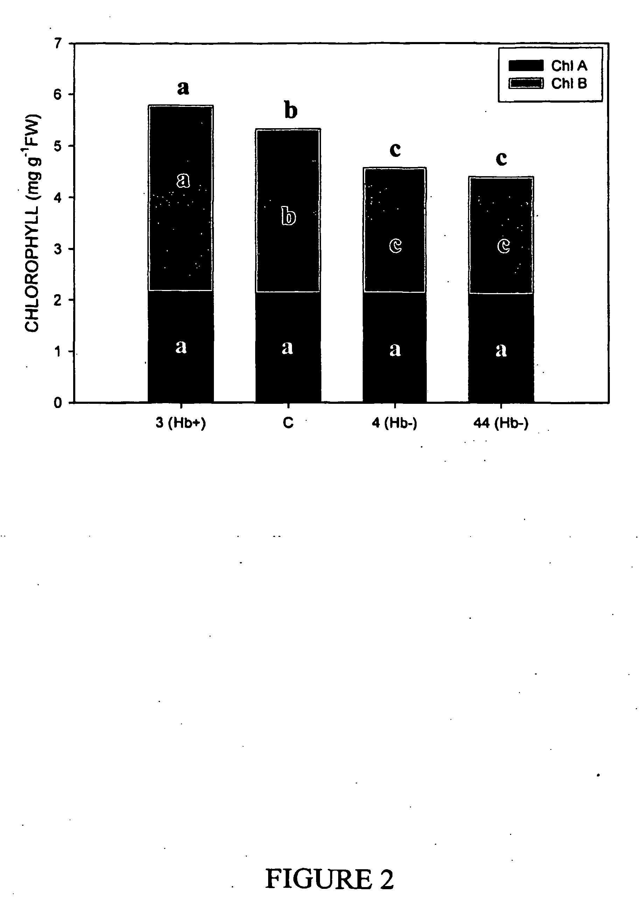

The modification of nsHb levels resulted in differences in leaf greenness and chlorophyll (chl) content, as shown in Fig. 2. Total chl content of nsHb+ plants was elevated relative to control plants whereas that of nsHb- plants was diminished. Changes in total chl content and Chl a:b ratios in transgenic lines were brought about solely by changes in Chl b, and not Chl a.

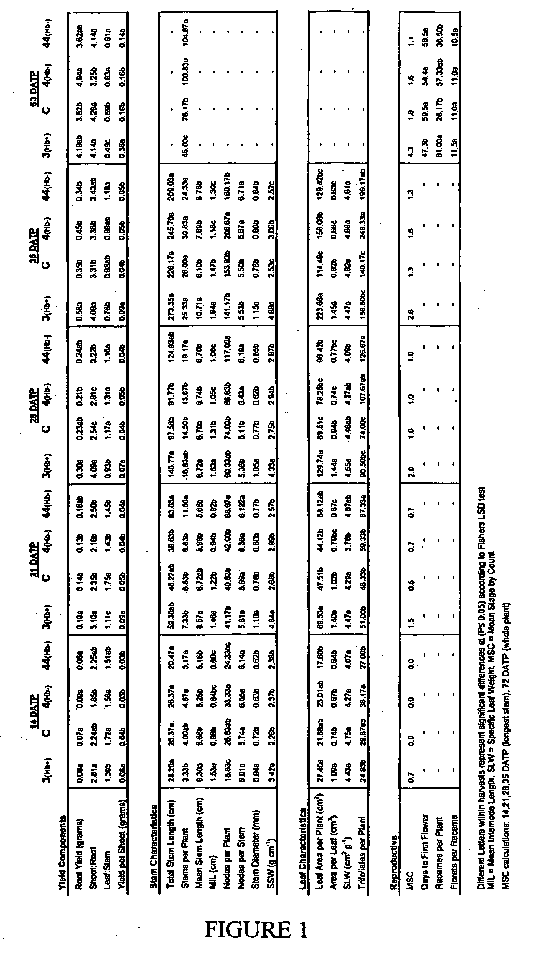

Figure 1 sets forth additional data for the four plant lines at 14, 21, 28, 35 and 63 DATP, discussed below.

### Yield, Stem, Leaf & Reproductive Characteristics

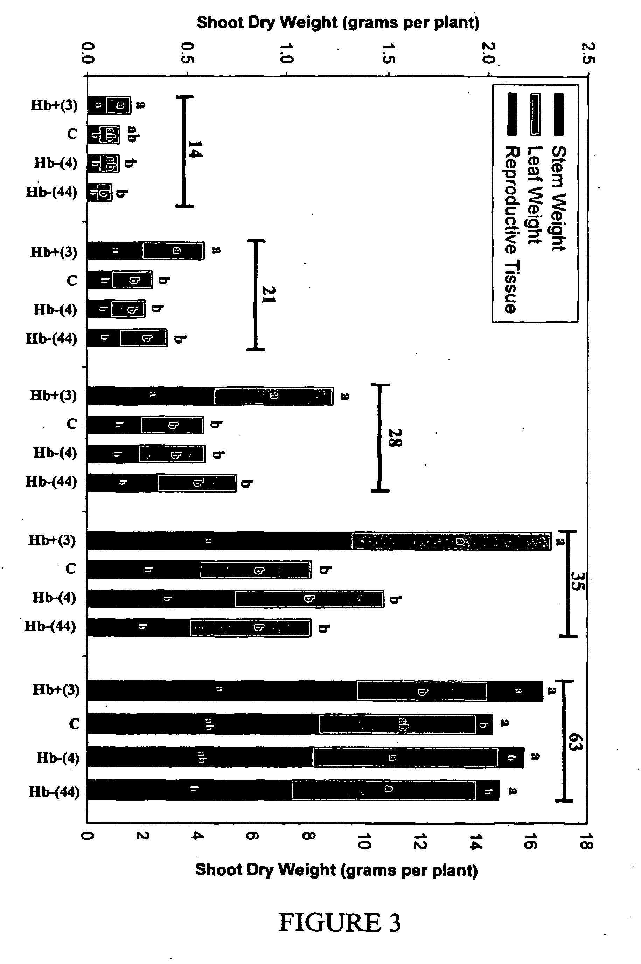

During early vegetative growth (14 to 35 DATP), nsHb+ plants produced 32-111% more shoot yield than control plants and produced significantly more shoot yield than all nsHb- plants across these same harvest dates. See Fig. 1 & Fig. 3. Adventitious root formation and resumption of shoot growth in nsHb⁻ lines in conjunction with high shoot to root ratios in nsHb⁺ lines led to non-significant differences in root yield of the transgenic lines at 14 DATP

For harvests occurring 21, 28 and 35 DATP, root yield of nsHb+ plants tended to follow herbage yield exceeding both control and nsHb- plants (Fig. 1). However, root yield of nsHb+ plants was significantly greater (P ≤ 0.05) than both control and nsHb-plants for the 35 DATP harvest only.

During vegetative growth, the shoot:root ratio of nsHb+ plants always exceeded that of control and nsHb- plants (Fig. 1). For all lines, the shoot:root ratio was observed to increase with successive harvests between 14 and 35 DATP (Fig. 1). For the 28 DATP harvest nsHb- (44) plants, which had slightly higher shoot yields (Fig. 3), also demonstrated elevated shoot:root ratios relative to control and nsHb- (4) plants (Fig. 1).

At the 63 DATP harvest, as was the case for harvests 14, 21, 28, and 35 DATP, nsHb+ plants consistently demonstrated a lower leaf:stem ratio than control and nsHb- plants (Fig. 1). The leaf:stem ratio of control and nsHb- plants did not significantly differ during vegetative growth except at 21 DATP where the leaf:stem ratio of control plants exceeded that of both nsHb+ and nsHb- plants (Fig. 1). At 63 DATP, the leaf stem ratio of nsHb+ plants was significantly reduced, and that of nsHb- plants significantly elevated, relative to control plants (Fig. 1).

For all harvest dates, yield per shoot (YPS) of nsHb+ plants exceeded that of control and nsHb- plants with no significant differences observed between control and nsHb-plants across all harvest dates (Fig. 1).

Stem weight of nsHb+ plants exceeded all other lines during vegetative growth. See Fig. 3. For these same harvest dates, leaf weight displayed a similar pattern to that of stem weight, except for the 14 DATP harvest where nsHb+ plants exceeded only nsHb- (44) plants for leaf weight. See Fig. 3. Leaf weight of control and nsHb- (4) plants was intermediate to that of nsHb+ and nsHb- (44) plants 14 DATP (Fig. 3). At the 63 DATP harvest, total shoot yield (leaf + stem + reproductive) was not significantly different between transgenic plants. However, nsHb+ plants were noted to flower sooner (see Fig. 1) and produce more reproductive tissue than control or nsHb- plants (see Fig. 3). At this same harvest date, stem yield was observed to decrease, and leaf yield increase, as nsHb expression declined. See Fig. 3. Similar results for shoot DW, in addition to significant differences in the FW:DW ratio in the shoots of transgenic lines, were observed in subsequent experiments, as shown in Table 2.

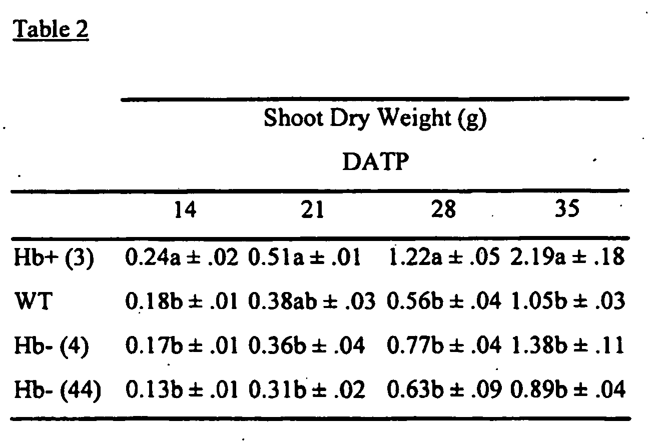

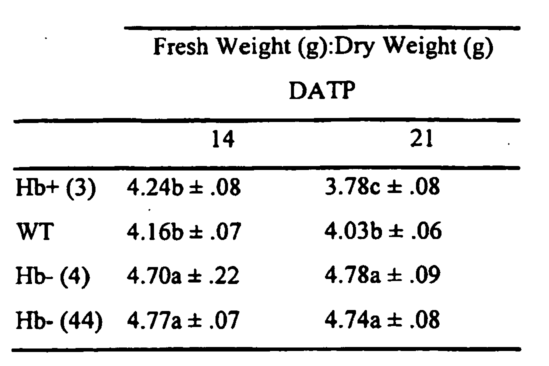

Table 2 shows shoot yield and fresh weight:dry weight ratios of transgenic alfalfa plants expressing varying levels of a class 1 nonsymbiotic barley hemoglobin. Different letters within harvest dates represent significant differences according to Fisher's protected LSD (P ≤ .05). Harvest dates expressed in DATP (days after transplanting).

Despite relatively few significant differences in the shoot and root yield of control and nsHb- plants, a number of morphological parameters distinguished these plants from one another in addition to nsHb+ plants. During vegetative growth the mean internode length and area per leaflet was increased in nsHb+ plants relative to control plants (Fig 1). In contrast, nsHb- plants experienced reductions in both of these parameters relative to control plants (Fig 1). In the case of nsHb- plants, impaired stem elongation and leaflet expansion resulted in production of greater numbers of stems per plant, nodes per stem and leaflets per plant (Fig 1). Although nsHb+ plants produced stem numbers equal to or less than control and nsHb- plants, stems produced were consistently thicker and had elevated specific stem weights (SSW) relative to control and nsHb- plants (Fig 1). nsHb+ plants also produced elongated and needled leaflets with longer petioles and petiolules. In comparison, nsHb- plants produced compressed oval leaflets with shortened petioles and petiolules.

Staging transgenic plants according to the MSC method of Kalu and Fick (1981) suggested nsHb+ plants to have accelerated morphological development relative to control and nsHb- plants at all harvest dates (Fig. 1). At 63 DATP, morphological development according to the MSC method placed nsHb- plants behind control plants. However, at this time Point nsHb- plants had more stems in the early (5) to late flower (6) stages than comparable control plants (data not shown), and lower MSC rankings were attributed to an extremely high number of stems in the early vegetative (0) stage. Greater weight of reproductive tissue and racemes per plant for nsHb- plants relative to control plants, although not significant, lend support to such observation (Fig. 1). As several nsHb+ plants contained stems in the late flower (6) and early seed pod (7) stages at 63 DATP, petal drop was suspected in having led to underestimation of reproductive tissue in this line.

### Root Morphology

Rooted stem cuttings and seedling root systems of transgenic alfalfa plants displayed a number of morphological characteristics which distinguished such plants from one another. nsHb expression appeared to influence the time required for cuttings to root, in addition to the number of adventitious roots forming on such cuttings. Cuttings of nsHb- and control plants rooted sooner than comparable cuttings from nsHb+ plants. nsHb- plants were observed to produce significantly more adventitious roots than nsHb+ or control plants 15 and 20 days after placing cuttings into rooting medium.

Table 3 sets forth data on shoot weight and root weight and characteristics for the four plant lines at 14, 21, 28, 35 and 63 DATP.

**Table 3**

| | Shoot Weight | Root Weight | MSC | TL | TD | DTP | LRN | LRP |
|---|---|---|---|---|---|---|---|---|
| Line | (grams) | (grams) | | (cm) | ------------------score^{z}------------------ | | | |
| Hb+ (3) | 3.63 | 0.65 | 1.97 | 63.10 | 4.83 | 0.17 | 1.50 | 2.83 |
| C | 2.45 | 0.26 | 0.31 | 65.08 | 3.17 | 1.00 | 1.67 | 3.33 |
| Hb- (4) | 2.42 | 0.17 | 0.14 | 52.22 | 2.33 | 2.00 | 2.00 | 5.00 |
| Hb- (44) | 2.13 | 0.17 | 0.22 | 45.50 | 1.67 | 2.67 | 2.33 | 5.00 |
| LSD_{(0.05)} | 1.41 | 0.31 | - | 16.97 | 0.68 | 1.15 | 0.77 | 1.57 |
| CV (%) | 43.93 | 81.39 | - | 24.95 | 18.75 | 65.34 | 34.08 | 32.33 |

When compared to control plants, roots of nsHb+ seedlings rapidly grew to the bottom of PVC pots and produced thicker, but not longer, taproots. (Table 3). In contrast, nsHb- plants allocated the majority of their root weight and root length to the upper portion of the soil strata, producing thin taproots slightly shorter than observed for control plants (Table 2). Non-significant differences in taproot length between lines were attributed to root growth of control and nsHb- lines along the soil-PVC interface and nsHb+ plants having reached the bottom of cylinders by 63 DATP.

Determinate taproot position (DTP) was used to gauge apical dominance of taproots below the crown of transgenic alfalfa plants. DTP score decreased with nsHb expression suggesting apical dominance of taproots to be lost as nsHb expression declined (Table 3) nsHb+ plants produced significantly fewer lateral roots than nsHb- (44) plants only, with control and nsHb-(4) plants falling intermediate (Table 3). Scoring transgenic plants for lateral root position (LRP), the lateral root closest to the crown, suggested nsHb- plants to position lateral roots closer to the crown than control and nsHb+ plants (Table 3). The differences in taproot diameter (Table 3) and fibrous root mass observed between root systems also resulted in significant differences in the specific root length (SRL) of transgenic lines. For all lines, SRL increased with soil depth. SRL of nsHb+ plants exceeded that of control plants, which in turn, exceeded nsHb- plants.

A notable characteristic of nsHb+ plants was an abundance of hypertrophied lenticels upon both taproots and lateral roots.

### Nutrient Uptake

Table 4 shows the mineral nutrient concentration in the shoots of the transformed alfalfa plants harvested 35 DATP (Days After Transplanting).

**Table 4**

| Shoots | | | | | | | |
|---|---|---|---|---|---|---|---|
| Nutrient | Hb+(3) | | C | | Hb-(4) | | Hb-(44) |
| N | 3.48a ±.13 | | 3.43a ±.11 | | 2.78b ± .13 | | 2.60b ± .12 |
| P | 0.25d ± .005 | | 0.31b ± .006 | | 0.28c ± .004 | | 0.33a ± .007 |
| K | 2.58a ± .06 | | 1.98b ± .03 | | 1.95b ± 0.03 | | 1.88b ±.03 |
| S | 0.330a ± .01 | | 0.303a ± .01 | | 0.230b ± .01 | | 0.318a ± .02 |
| Ca | 2.033b ± .0.5 | | 3.235a ± .06 | | 3.008a ± .12 | | 3.163a ± .25 |
| Mg | 0.583b ± .0.1 | | 0.953a ± .02 | | 0.908a ± .04 | | 0. 940a ± .05 |
| Na | 0.033c ± .003 | | 0.035bc ± .003 | | 0.048b ± .003 | | 0.040b ± .003 |
| Zn | 15.75b ± .48 | | 23.75a ± 3.77 | | 19ab ± .41 | | 20.75ab ± .95 |
| Fe | 99.5a ± 9.79 | | 103.25a ± 3.12 | | 11 2.25a ± 10.42 | | 155.5a ± 50.47 |
| Mn | 95.25c ± 4.03 | | 164.25b ± 4.80 | | 149.75b ± 24.42 | | 258.75a ± 24.49 |
| Cu | 10.5c ± .29 | | 17.75b ± .25 | | 18.25b ± 1.44 | | 21.25a ± .75 |
| B | 65.5c ± 1.85 | | 97.25ab ± 1.60 | | 90.5b ± 3.80 | | 109a ± 8.22 |

The nutrient analysis for Nitrogen (N), Phosphorus (P), Potassium (K), Sulphur (S), Calcium (Ca), Magnesium (Mg) and Sodium (Na) is expressed on a % dry matter basis. The nutrient analysis for Zinc (Zn), Iron (Fe), Manganese (Mg), Copper (Cu), and Boron (B) is expressed in parts per million (ppm). The different letters (a, b, c, d) listed in the amounts represent significant differences according to Fisher's Protected LSD (P < .05).

## Claims

1. A method of modifying a plant phenotype, comprising:
transforming a plant with an expression vector comprising a nucleotide sequence encoding a plant non-symbiotic hemoglobin or an antisense sequence thereto, thereby yielding a transformed plant having an altered level of expression of non-symbiotic plant hemoglobin as compared to a non-transformed control plant that is not transformed to alter the level of expression of non-symbiotic plant hemoglobin,
wherein said transformed plant exhibits, under normal oxygen conditions, a plant phenotype that is modified as compared to said non-transformed control plant,
wherein said phenotype is selected from the group consisting of shoot or root apical dominance; flower color; and chlorophyll content
wherein, when said transformed plane exhibits an increased level of expression of non-symbiotic hemoglobin as compared to said non-transformed control plant, said plant exhibits increased shoot apical dominance or greater root apical dominance under normal oxygen conditions as compared to said non-transformed control plant.

2. The method of claim 1, wherein said transformed plant exhibits an increased level of expression of non-symbiotic hemoglobin as compared to said non-transformed control plant.

3. The method of claim 2, wherein said transformed plant exhibits increased shoot apical dominance under normal oxygen conditions as compared to said non-transformed control plant.

4. The method of claim 2, wherein said transformed plant exhibits reduced flower pigmentation under normal oxygen conditions as compared to said non-transformed control plant.

5. The method of claim 1, wherein said transformed plant exhibits a decreased level of expression of non-symbiotic hemoglobin as compared to said non-transformed control plant.

6. The method of claim 2, wherein said method comprises transforming said plant with an expression vector comprising a nucleic acid molecule encoding a plant non-symbiotic hemoglobin.

7. The method of claim 5, wherein said method comprises transforming said plant with an expression vector comprising an antisense plant non-symbiotic hemoglobin nucleic acid molecule.

8. The method of claim 1, wherein said expression vector comprises an inducible promoter that permits selective induction of expression of a plant non-symbiotic hemoglobin.

9. The method of claim 1, wherein said expression vector comprises a repressible promoter that permits selective repression of expression of a plant non-symbiotic hemoglobin.

## Patentansprüche

1. Verfahren zur Modifizierung eines Pflanzenphänotyps, umfassend:
Transformieren einer Pflanze mit einem Expressionsvektor, umfassend eine Nukleotidsequenz, die für ein pflanzliches nicht symbiotisches Hämoglobin oder eine Antisense-Sequenz davon kodiert, wodurch sich eine transformierte Pflanze ergibt, die im Vergleich zu einer nicht transformierten Kontrollpflanze, die nicht transformiert ist, um das Expressionsniveau von pflanzlichem nicht symbiotischem Hämoglobin zu ändern, ein verändertes Expressionsniveau von pflanzlichem nicht symbiotischem Hämoglobin aufweist,
wobei die transformierte Pflanze unter normalen Sauerstoffbedingungen einen Pflanzenphäntoyp zeigt, der im Vergleich zu der nicht transformierten Kontrollpflanze modifiziert ist,
wobei der Phänotyp ausgewählt ist aus der Gruppe, bestehend aus Apikaldominanz am Trieb oder an der Wurzel; Blumenfarbe und Chlorophyllgehalt,
wobei die Pflanze, wenn die transformierte Pflanze ein im Vergleich zu der nicht transformierten Kontrollpflanze erhöhtes Expressionsniveau von nicht symbiotischem Hämoglobin zeigt, unter normalen Sauerstoffbedingungen eine im Vergleich zu der nicht transformierten Kontrollpflanze verstärkte Apikaldominanz am Trieb oder eine erhöhte Apitaldominanz an der Wurzel zeigt.

2. Verfahren nach Anspruch 1, wobei die transformierte Pflanze ein im Vergleich zu der nicht transformierten Kontrollpflanze erhöhtes Expressionsniveau von nicht symbiotischem Hämoglobin zeigt.

3. Verfahren nach Anspruch 2, wobei die transformierte Pflanze unter normalen Sauerstoffbedingungen eine im Vergleich zu der nicht transformierten Kontrollpflanze verstärkte Apikaldominanz am Trieb zeigt.

4. Verfahren nach Anspruch 2, wobei die transformierte Pflanze unter normalen Sauerstoffbedingungen eine im Vergleich zu der nicht transformierten Kontrollpflanze geringere Blütenpigmentierung zeigt.

5. Verfahren nach Anspruch 1, wobei die transformierte Pflanze ein im Vergleich zu der nicht transformierten Kontrollpflanze geringeres Expressionsniveau von nicht symbiotischem Hämoglobin zeigt.

6. Verfahren nach Anspruch 2, wobei das Verfahren das Transformieren der Pflanze mit einem Expressionsvektor umfasst, der ein Nukleinsäuremolekül umfasst, das für ein pflanzliches nicht symbiotisches Hämoglobin codiert.

7. Verfahren nach Anspruch 5, wobei das Verfahren das Transformieren der Pflanze mit einem Expressionsvektor umfasst, der ein Antisense-Nukleinsäuremolekül für ein pflanzliches nicht symbiotisches Hämoglobin umfasst.

8. Verfahren nach Anspruch 1, wobei der Expressionssektor einen induzierbaren Promotor umfasst, der die selektive Induktion der Expression eines pflanzlichen nicht symbiotischen Hämoglobins ermöglicht.

9. Verfahren nach Anspruch 1, wobei der Expressionssektor einen reprimierbaren Promotor umfasst, der die selektive Repression der Expression eines pflanzlichen nicht symbiotischen Hämoglobins ermöglicht.

## Revendications

1. Méthode de modification d'un phénotype végétal, comprenant :
transformation d'un végétal à l'aide d'un vecteur d'expression comprenant une séquence nucléotidique codant pour une hémoglobine végétale non symbiotique ou une séquence antisens de celle-ci, produisant ainsi un végétal transformé présentant un niveau altéré d'expression d'hémoglobine végétale non symbiotique par comparaison à un végétal témoin non transformé qui n'a pas subi de transformation altérant le niveau d'expression d'hémoglobine végétale non symbiotique,
où ledit végétal transformé présente, dans des conditions d'oxygène normales, un phénotype végétal qui est modifié par comparaison au dit végétal témoin non transformé,
où ledit phénotype est choisi dans le groupe consistant en dominance apicale des pousses ou des racines ; couleur des fleurs et teneur en chlorophylle,
où, quand ledit végétal transformé présente un niveau accru d'expression d'hémoglobine non symbiotique par comparaison au dit végétal témoin non transformé, ledit végétal présente une dominance apicale des pousses accrue ou une dominance apicale des racines accrue dans des conditions d'oxygène normales par comparaison au dit végétal témoin non transformé.

2. La méthode de la revendication 1, où ledit végétal transformé présente un niveau accru d'expression d'hémoglobine non symbiotique par comparaison au dit végétal témoin non transformé.

3. La méthode de la revendication 2, où ledit végétal transformé présente une dominance apicale des pousses accrue dans des conditions d'oxygène normales par comparaison au dit végétal témoin non transformé.

4. La méthode de la revendication 2, où ledit végétal transformé présente une pigmentation florale réduite dans des conditions d'oxygène normales par comparaison au dit végétal témoin non transformé.

5. La méthode de la revendication 1, où ledit végétal transformé présente un niveau réduit d'expression d'hémoglobine non symbiotique par comparaison au dit végétal témoin non transformé.

6. La méthode de la revendication 2, où ladite méthode comprend la transformation dudit végétal à l'aide d'un vecteur d'expression comprenant une molécule d'acide nucléique codant pour une hémoglobine végétale non symbiotique.

7. La méthode de la revendication 5, où ladite méthode comprend la transformation dudit végétal à l'aide d'un vecteur d'expression comprenant une molécule d'acide nucléique d'hémoglobine végétale non symbiotique antisens.

8. La méthode de la revendication 1, où ledit vecteur d'expression comprend un promoteur inductible permettant une induction sélective de l'expression d'une hémoglobine végétale non symbiotique.

9. La méthode de la revendication 1, où le dit vecteur d'expression comprend un promoteur répressible permettant une répression sélective de l'expression d'une hémoglobine végétale non symbiotique.
